# EUROPEAN PATENT SPECIFICATION

(11) **EP 1 414 571 B1**
(45) Date of publication and mention of the grant of the patent: **31.03.2010**
(21) Application number: 02734550.3
(22) Date of filing: 29.05.2002
(51) Int. Cl.: B01L 3/00

(54) **WELL FOR PROCESSING AND FILTERING A FLUID**
KAVITÄT ZUM PROZESSIEREN UND FILTERN VON FLUIDEN
PUITS SERVANT A TRAITER UN FLUIDE

(30) Priority: 31.05.2001 US 294211 P
(43) Date of publication of application: 06.05.2004
(60) Divisional application: 09178039.5
(73) Proprietor: Pall Corporation, Port Washington, NY 11050 (US)
(72) Inventor: KANE, Jeffrey, Manchester, MI 48158 (US)
(74) Representative: Hoeger, Stellrecht & Partner Patentanwälte
(86) International application number: PCT/US2002/016686
(87) International publication number: WO 2002/096563

(56) References cited:
- EP-A- 0 359 249
- EP-A- 0 403 679
- EP-A- 1 110 611
- DE-A- 4 114 611
- US-A- 4 170 056
- US-A- 4 948 442
- US-A- 4 948 564
- US-A- 5 047 215
- US-A- 5 918 273
- US-A- 6 159 368

## Description

### FIELD OF THE INVENTION

This invention relates to wells for use in processing fluids, e.g.; by titration and/or filtration, and more preferably relates to multiwell microtitration and microfiltration devices.

### BACKGROUND OF THE INVENTION

Multiple well test devices are used in a variety of microtitration and/or microfiltration protocols. Typically, the devices have a standard number of wells, e.g., 96 wells, or 384 wells (arranged in four blocks of 96 wells each), and the wells include a filter arranged such that application of a vacuum or air pressure to one side of the device causes the fluid in each well to pass through the filter.

When the device is used in for microtitration, e.g., for an immunoassay or a hybridization assay, the filter is generally used to support one or more components of the assay, such as an antigen, antibody, nucleic acid probe or nucleic acid sample. A supported component (e.g., a specific binding agent) binds to an unsupported component in a fluid sample. At least one component can be contained in, on, and/or through the filter, e.g., by physical entrapment, chemical binding and/or adsorption.

When used for microfiltration the filter is generally used to remove one or more components from solutions passed through it, e.g., on the basis of physical, biological and/or chemical interactions in or on the filter. The interactions can be, for example, between the filter and the component to be retained, or between one or more materials retained in or on the filter and the component to be retained.

Accordingly, the devices can be utilized to remove one or more undesirable and/or desirable materials from a fluid. For example, the devices can be used to remove particulates from a sample before further processing (e.g., analysis) of the sample and/or to retain a desired ligand for later recovery and further processing.

Some conventional devices are labor intensive to manufacture and/or are not adapted for use with a variety of filters. Additionally, the seal of the filter in the device can be adversely affected by handling and/or fluid processing conditions.
EP 0 403 679 is directed to a multi-well filtration apparatus for the assay of microliter quantities of biological and biochemical reactants. A filter is positioned in each well on a plate having an open spout having a collar which prevents a liquid droplet from climbing the outer surface of the spout from its open end.
US Patent No. 4,170,056 is directed to a blood filter including a stack of filter pads positioned within a housing. The stack of the filter pads may be joined together at their periphery by a heat seal and joined to the housing at the periphery by a second seal.

The present invention provides for ameliorating at least some of the disadvantages of the prior art. These and other advantages of the present invention will be apparent from the description as set forth below.

### BRIEF SUMMARY OF THE INVENTION

In accordance with an embodiment of the invention, a well for processing fluid is provided, the well comprising a hollow tube comprising a side wall having an inner surface, a bottom end comprising a bottom wall and a fluid flow port, and a circumferential rib projecting upwardly from the bottom wall, the rib having a top surface spaced away from the inner surface of the side wall; a sealing ring having an outer surface and a bottom surface, the outer surface of the sealing ring pressing against the inner surface of the side wall; and a filter comprising at least one filter element, the filter having an upper surface and a lower surface, the filter being compressed between the bottom surface of the sealing ring and the top surface of the rib. Typically, the bottom surface of the sealing ring presses against the upper surface of the filter while the top surface of the rib presses against the lower surface of the filter. In a preferred embodiment of the well, the bottom surface of the sealing ring has a width that is at least the width of the top surface of the rib arrangement.

In some embodiments of the well, the bottom end also includes a drainage grid comprising at least one drainage channel communicating with the fluid flow port. For example, the bottom end can include a drainage grid comprising a plurality of drainage grid spacers projecting upwardly from the bottom wall, wherein a plurality of drainage channels are provided between the drainage grid spacers, and the drainage channels communicate with the fluid flow port.

In accordance with another embodiment, a plurality of the wells are connected together to provide a multiple well device, e.g., in the form of a 96 or 384 well device, in some embodiments, a 96 or 384 well tray.

Methods are also provided for processing a fluid utilizing embodiments of the well and the multiple well devices. Suitable methods include, for example, microtitration, microfiltration, and microculture procedures.

### BRIEF DESCRIPTION OF THE DRAWINGS

Figure 1 shows a top view of an embodiment of a base suitable for use in a multiple well device according to the present invention, showing a first well including filter and a sealing ring, a second well including a rib arrangement and fluid flow port, and a third well including a rib arrangement, a drainage grid, and a fluid flow port.

Figure 2 shows a partial cross-sectional view of a portion of the base including the third well shown in Figure 1, showing the inner surface of the side wall of the well, the bottom wall of the well, as well as the rib arrangement and the drainage grid projecting upwardly from the bottom wall.

Figure 3 shows an enlarged top view of the third well shown in Figure 1, the well including the rib arrangement, the drainage grid, and the fluid flow port.

Figure 4 shows an embodiment of a sealing ring. Figure 4a shows a top view, and Figure 4b shows a cross-section view along lines 4b-4b in Figure 4a.

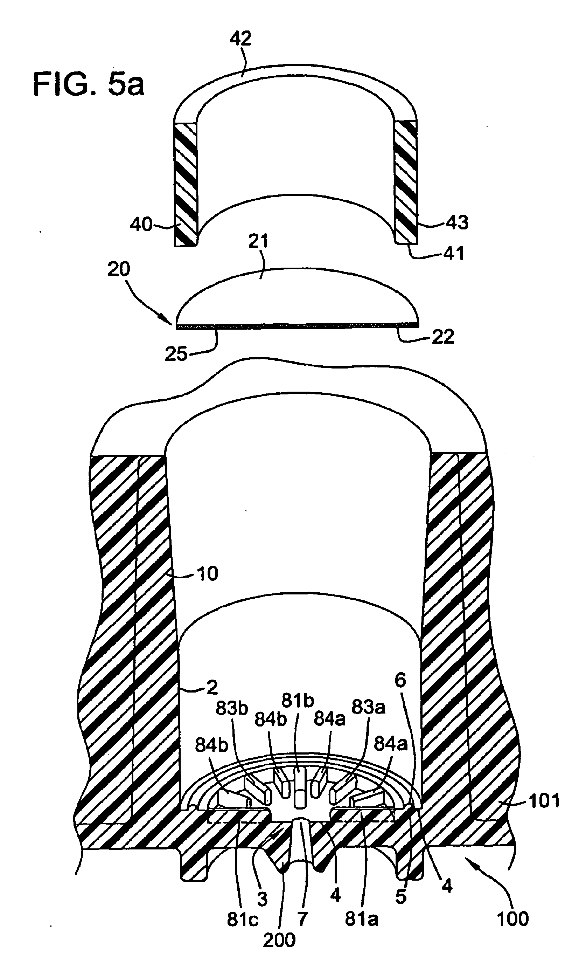

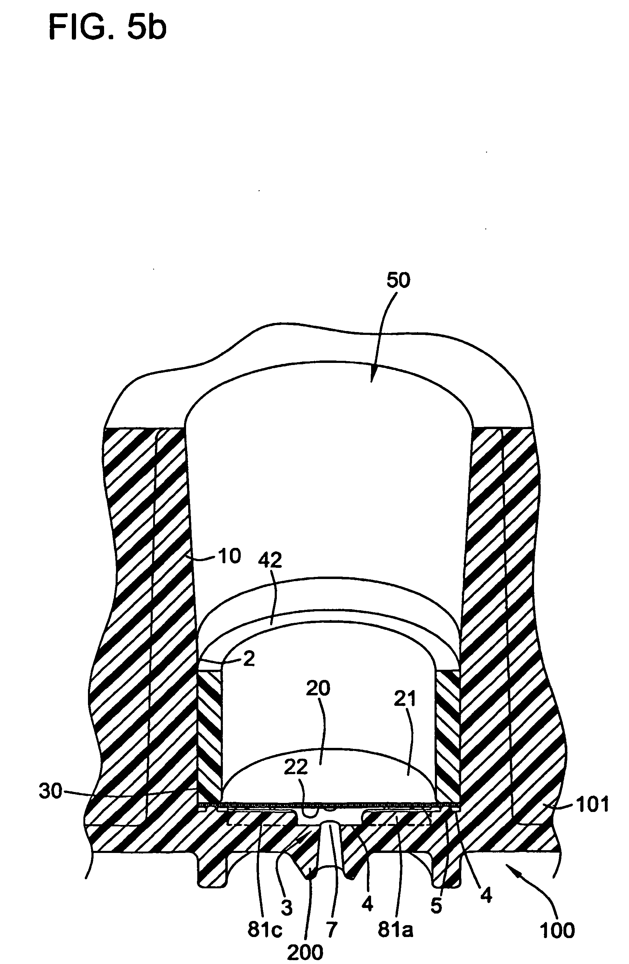

Figure 5 shows partial cut away views of a portion of a well according to an embodiment of the invention, showing a sealing ring, a filter, a rib arrangement, and a drainage grid. Figure 5a shows an exploded view, and Figure 5b shows an assembled view wherein the sealing ring is retained such that the outer surface of the sealing ring presses against the inner surface of the side wall of the well, and the bottom surface of the ring presses against the upper surface of the filter while the top surface of the rib arrangement presses against the lower surface of the filter so that the filter is sealed in the well.

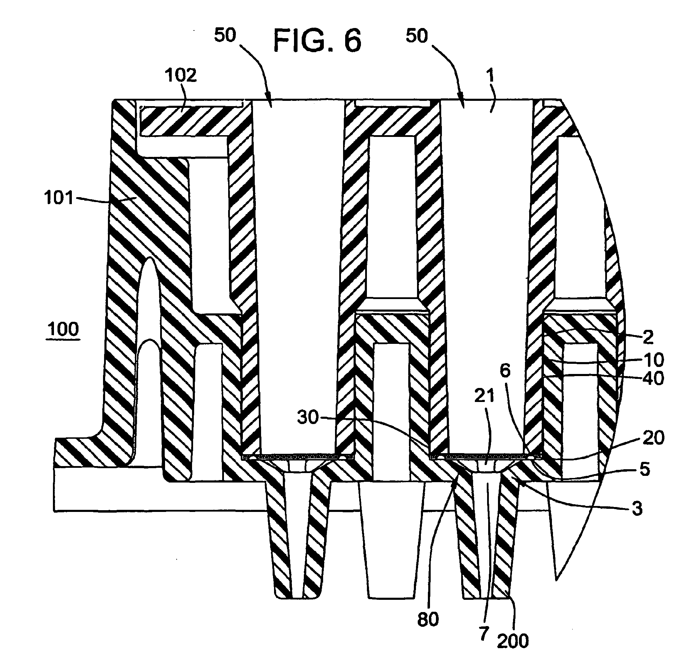

Figure 6 shows a partial cross-sectional view of another embodiment of a multiple well device according to the invention, showing a top plate including a plurality of sealing rings, and a bottom plate including the bottom ends of a plurality of wells, wherein each end includes a bottom wall, a fluid flow port, and a rib arrangement projecting upwardly from the bottom wall, herein the sealing ring is retained such that the outer surface of the sealing ring presses against the inner surface of the side wall of the well, and the bottom surface of the ring presses against the upper surface of the filter while the top surface of the rib arrangement presses against the lower surface of the filter so that the filter is sealed in the well.

Figure 7 shows a top view of the bottom plate shown in Figure 6, showing the bottom ends of a plurality of wells, wherein each end includes a bottom wall, a fluid flow port, and a rib arrangement projecting upwardly from the bottom wall.

Figure 8 shows a top view of the bottom plate including the bottom end of one of the wells shown in Figure 6, showing a bottom wall, a rib arrangement, and a fluid flow port.

Figure 9 shows a top view of the top plate shown in Figure 6.

Figure 10 shows partial cross-sectional views of the plates shown in Figure 6. Figure 10a shows a partial cross-sectional view of the top plate, and Figure 10b shows a partial cross-sectional view of the bottom plate.

### DETAILED DESCRIPTION OF THE INVENTION

In one embodiment of the present invention, a well for use in processing a fluid comprises a hollow tube having an axis, the tube comprising a side wall having an inner surface; upper and lower axially spaced ends, the lower end comprising a bottom wall and a fluid flow port, and a circumferential rib projecting upwardly from the bottom wall, the rib having a top surface spaced from the inner surface of the side wall; a sealing ring having an outer surface and a bottom surface, the outer surface of the sealing ring pressing against the inner surface of the side wall; and a filter comprising at least one filter element, the filter having a upper surface and a lower surface, the filter being compressed between the bottom surface of the sealing ring and the top surface of the rib arrangement. Preferably, the bottom surface of the sealing ring has a radial width that is at least the radial width of the top surface of the rib arrangement. Typically, the sealing ring seals the filter against the rib wherein the bottom surface of the sealing ring presses against the upper surface of the filter while the top surface of the rib presses against the lower surface of the filter. In some embodiments, the sealing ring has a hollow bore suitable for receiving fluid, wherein the bore substantially extends to the upper end of the tube.

A well for use in processing a fluid according to another embodiment of the invention comprises a hollow tube having an axis, the tube comprising a side wall having an inner surface; upper and lower axially spaced ends, the lower end comprising a bottom wall, a fluid flow port, a drainage grid arrangement comprising at least one drainage grid spacer projecting upwardly from the bottom wall, and a circumferential rib projecting upwardly from the bottom wall, the rib arrangement having a top surface spaced from the inner surface of the side wall of the tube; a filter comprising at least one filter element, the filter having an upper surface and a lower surface; and a sealing ring sealing the filter against the rib, the sealing ring having an outer surface, and a bottom surface, the bottom surface having a radial width that is at least the radial width of the top surface of the rib, the outer surface of the sealing ring pressing against the inner surface of the side wall of the tube, and the bottom surface of the ring pressing against the upper surface of the filter while the top surface of the rib presses against the lower surface of the filter.

In accordance with another embodiment, a microtitration or microfiltration device is provided comprising a well comprising a hollow tube having an axis, the tube comprising a side wall having an inner surface, upper and lower axially spaced ends, the lower end comprising a bottom wall, a drainage grid comprising at least one drainage channel, and a fluid flow port, and a circumferential rib projecting upwardly from the bottom wall, the rib arrangement having a top surface that does not contact the inner surface of the side wall of the tube, a filter comprising at least one filter element, the filter having an upper surface and a lower surface, and a sealing ring sealing the filter against the rib, the sealing ring having an outer surface, and a bottom surface, the bottom surface having a width that is at least the width of the top surface of the rib, the outer surface of the sealing ring pressing against the inner surface of the side wall of the tube, and the bottom surface of the ring pressing against the upper surface of the filter while the top surface of the rib presses against the lower surface of the filter.

In accordance with another embodiment, a device for culturing a cell suspension or tissue comprises a well for receiving a cell suspension or tissue, the well having a side wall having an inner surface, a bottom end comprising a bottom wall and a fluid flow port, and a circumferential rib projecting upwardly from the bottom wall, the rib having a top surface that does not contact the inner surface of the side wall; a filter sealed in the well, the filter comprising at least one filter element suitable for culturing the cell suspension or tissue, the filter having a upper surface and a lower surface; and a sealing ring sealing the filter against the rib, the sealing ring having an outer surface, and a bottom surface, the bottom surface having a width that is at least the width of the top surface of the rib the sealing ring pressing against the inner surface of the side wall, and the bottom surface of the ring pressing against the upper surface of the filter while the top surface of the rib presses against the lower surface of the filter. Typically, device further comprises a culture medium contained in the Well.

In more preferred embodiments of the invention, a plurality of wells are connected together to provide a multiple well device. In accordance with embodiments of the invention, the potential for cross-contamination from one well to another can be eliminated.

An embodiment of a multiple well device according to the invention comprises a plurality of wells for receiving liquid samples to be processed, each well having a side wall having an inner surface, a bottom end comprising a bottom wall and a fluid flow port, and a circumferential rib projecting upwardly from the bottom wall, the rib having a top surface spaced from the inner surface of the side wall; a sealing ring having an outer surface, and a bottom surface, the outer surface of the sealing ring pressing against the inner surface of the side wall of the tube; and a filter comprising at least one filter element, the filter having a upper surface and a lower surface, the filter being compressed between the bottom surface of the sealing ring and the top surface of the rib.

In another embodiment, a multiple well device comprises a plurality of wells for receiving liquid samples to be processed, each well comprising a side wall having an inner surface, a bottom end comprising a bottom wall and a fluid flow port, and a circumferential rib projecting upwardly from the bottom wall, the rib having a top surface that does not contact the inner surface of the side wall; a filter comprising at least one filter element, the filter having a upper surface and a lower surface; and a sealing ring sealing the filter against the rib, the sealing ring having an outer surface and a bottom surface, the bottom surface having a width that is at least the width of the top surface of the rib, the outer surface of the sealing ring pressing against the inner surface of the side wall, and the bottom surface of the ring pressing against the upper surface of the filter while the top surface of the rib presses against the lower surface of the filter.

In some embodiments of multiple well devices according to the invention, the device comprises at least a top plate and a bottom plate, wherein the top plate comprises the sealing ring, and the bottom plate comprises the bottom wall and the circumferential rib. In one embodiment wherein the top plate comprises the sealing ring, the sealing ring has a hollow bore that substantially extends to the top surface of the plate.

Embodiments of multiple well devices include wells connected together in the form of, for example, a strip, disc, or tray. Multiple well devices can include integrally formed wells (e.g., formed by injection or blow molding), or the wells can be formed from a plurality of components, e.g., an upper plate, tray or insert defining the upper portion of the wells and/or the sealing ring, and a bottom plate, tray or insert defining at least the bottom wall of the wells.

The wells, and more preferably, the multiple well devices, are suitable for use with a variety of other components such as a chamber, plenum, manifold, manifold cover, or the like, e.g., for applying reduced pressure or vacuum to the wells. Accordingly, some embodiments of the wells and multiple well devices further comprise, for example, a manifold cover.

Embodiments of methods for using the wells and multiple well devices are also provided.

For example, one embodiment of a method for processing a fluid comprises passing a fluid into a well comprising a side wall having an inner surface, and a bottom end having a circumferential rib having a top surface, wherein the top surface of the rib is spaced from the inner surface of the side wall, and a fluid flow port; the well having a filter comprising at least one filter element sealed therein, the filter being compressed between a lower surface of a sealing ring, and the top surface of the rib, and, passing at least a portion of the fluid through the filter and through the fluid flow port at the bottom end of the well.

Preferably, the method comprises processing fluid in a multiple well device, wherein fluid is passed into a plurality of wells as described above.

Embodiments of the method can include binding at least one component in the fluid to be processed to at least one binding agent in the well, in some embodiments, binding at least one component in the fluid to be processed to at least one binding agent in or on the filter. For example, embodiments of the method can include antigen-antibody binding, binding a nucleic acid in a sample to a complementary nucleic acid probe, binding ligands, and binding proteins. In some embodiments, at least one binding agent is a specific binding agent such as a monoclonal antibody or a nucleic acid probe that specifically binds to a component in the fluid to be processed. Other embodiments of the method include, for example, removing particulates from the fluid to be processed, and synthesizing desired materials in the wells. If desired, embodiments of the method also include analyzing the filtrate passing through the filter and/or analyzing the bound component (in some embodiments, after cleaving the bound component from the well).

Each of the components of the invention will now be described in more detail below, wherein like components have like reference numbers.

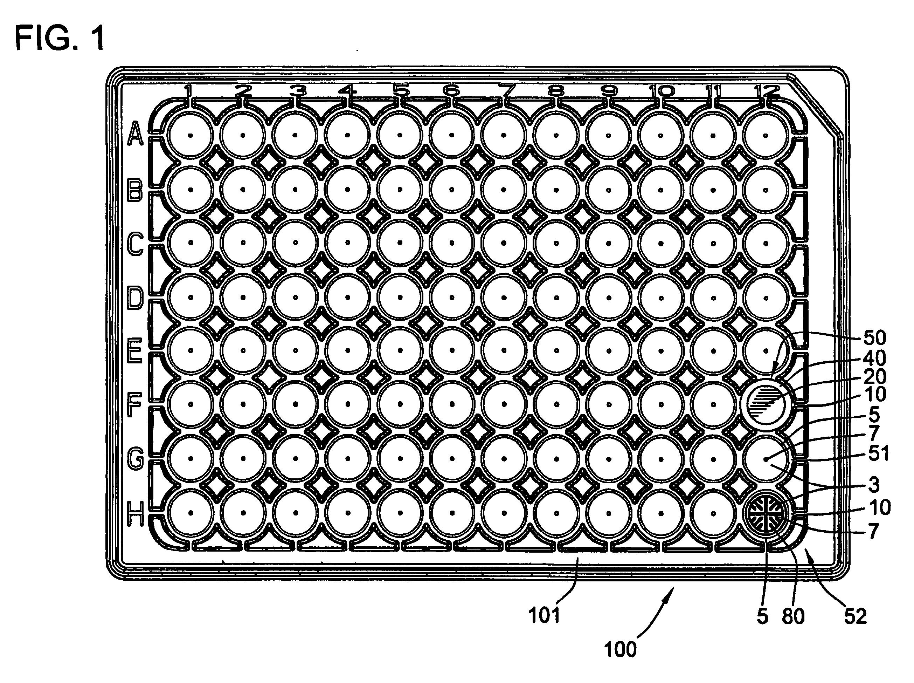

Figure 1 shows a top view of a multiple well device 100 according to an embodiment of the invention, comprising a base or bottom plate 101 suitable for providing the bottom ends for a plurality of wells. Figure 1 also shows an assembled well 50 according to an embodiment of the invention, as well as two non-assembled wells 51 and 52. Assembled well 50 comprises a side wall 10, a sealing ring 40, and a filter 20. While not shown in assembled well 50 the two non-assembled wells 51 and 52 show a bottom end 3, comprising a circumferential rib 5 and a fluid flow port 7 (all covered by filter 20 in the assembled well 50), and a side wall 10. Non-assembled well 52 also shows a drainage grid 80, as well as the rib arrangement 5.

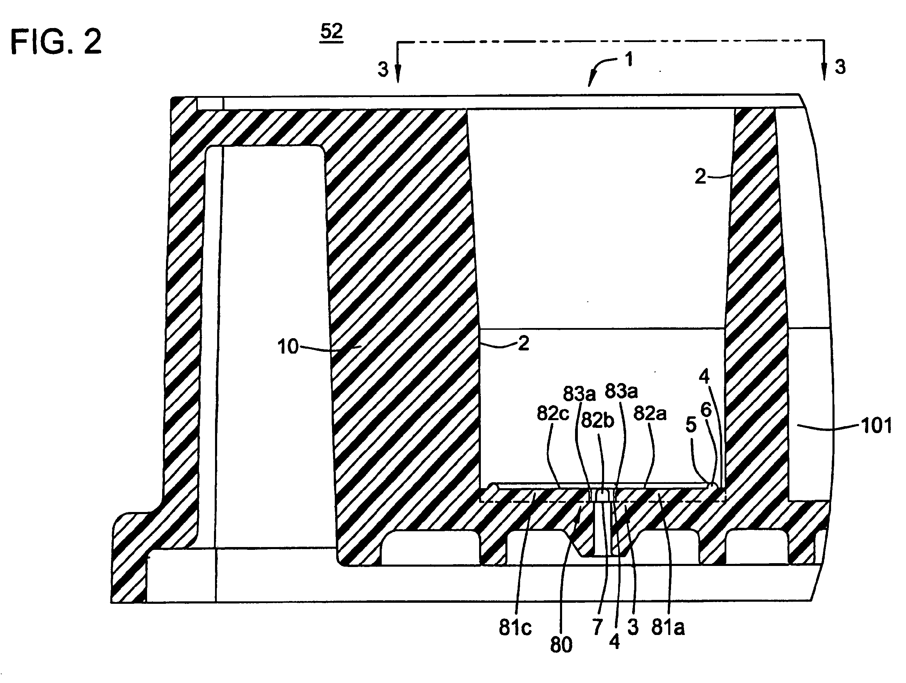

Figure 2 shows a partial cross-sectional view of a portion of the bottom plate 101 including the non-assembled well 52 shown in Figure 1, showing the side wall 10 haying an inner surface 2, and the bottom end 3, comprising a bottom wall 4, a circumferential rib 5 projecting upwardly from the bottom wall, the rib having a top surface 6, a drainage grid 80, and a fluid flow port 7.

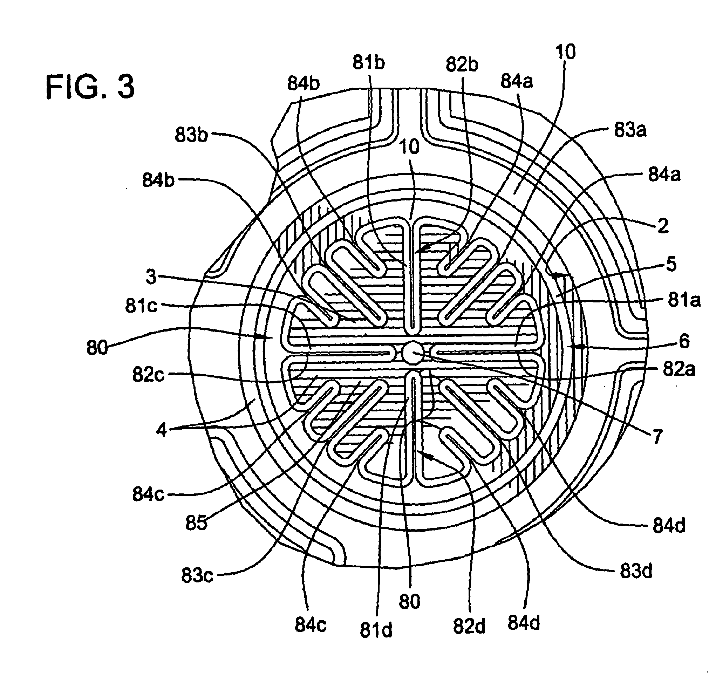

Figure 3 shows a top view of the non-assembled well 52 shown in Figure 2, showing the side wall 10 having an inner surface 2, and the bottom end 3, comprising a bottom wall 4, a circumferential rib 5 having a top surface 6, a drainage grid 80, and a fluid flow port 7.

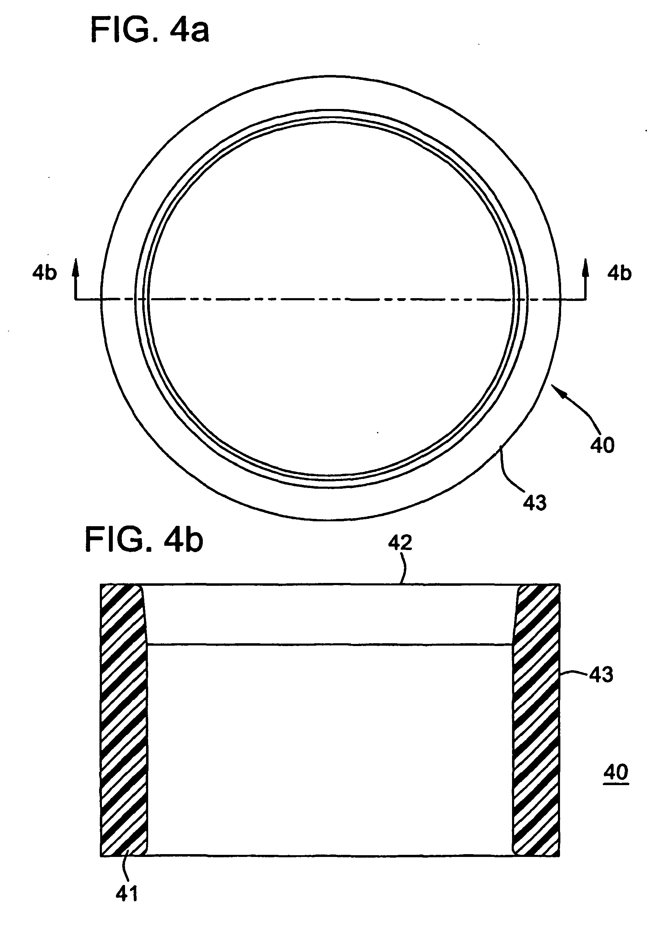

Figure 4 shows an embodiment of a sealing ring 40. Figure 4a shows a top view of sealing ring 40 having an outer surface 43, and Figure 4b shows a cross-section view along lines 4b-4b in Figure 4a, showing the top surface 42, the bottom surface 41, and the outer surface 43 of sealing ring 40.

The separate and/or partially assembled components shown in Figures 1-4 are shown arranged in Figures 5a (exploded view) and 5b (assembled view), that illustrate a partial cut away view of a device comprising a well according to an embodiment of the invention. Using Figures 5a and 5b for reference, the illustrated device 100 comprises a well 50 comprising a hollow tube, and a filter 20 having an upper surface 21 and a lower surface 22 (the filter comprising a filter elements 25), interposed between a sealing ring 40 having a bottom surface 41, and a circumferential rib 5 having an top surface 6, wherein the lower surface 22 of the filter contacts the top surface 6 of the rib, and the upper surface 21 of the filter contacts the bottom surface 41 of the sealing ring. The hollow tube comprises a side wall 10 having an axis, the side wall 10 having an inner surface 2, the well having a top (or upper) end 1 and a bottom (or lower) end 3 wherein the ends are axially spaced, the bottom end 3 comprising a bottom wall 4 and a fluid flow port 7, and a circumferential rib 5 projecting upwardly from the bottom wall

In the embodiment shown in Figure 5b, the circumferential rib 5 has a raised portion such as a rib, ridge, or boss, and has a top surface 6 (that is preferably a non-planar surface, e.g., a rounded surface) disposed to contact the lower surface 22 of a filter 20 comprising at least one filter element 25.

The top surface 6 of the rib illustrated in Figures 5a and 5b does not contact the inner surface 2 of the side wall of the well (the Figures show a depression between the top surface of the rib and the inner surface of the side wall), and the circumferential rib can be located a predetermined distance from the inner surface of the side wall. In this illustrated embodiment, a sealing ring 40 having a bottom surface 41, a top surface 42, and an outer surface 43, is retained against the inner surface of the hollow tubular wall, the outer surface 43 of the ring pressing against the inner surface 2 of the side wall (e.g., at location 30, that can be a predetermined position along the inner surface of the wall), and the lower surface 41 of the ring pressing against the upper surface 21 of the filter 20 while the top surfaces of the rib 5 presses against the lower surface 22 of the filter. The sealing ring 40 efficiently seals the filter 20 in the well, typically by compressing the portion of the filter surface contacting the sealing ring lower surface 41 as the portion of the filter surface contacting top surface 6 is also compressed. Thus, fluid is prevented from bypassing the filter (i.e., passing around the edges of the filter rather than through the filter). Accordingly, fluid passes from the upper surface of the filter through the lower surface of the filter, and passes from the well through, flow port 7.

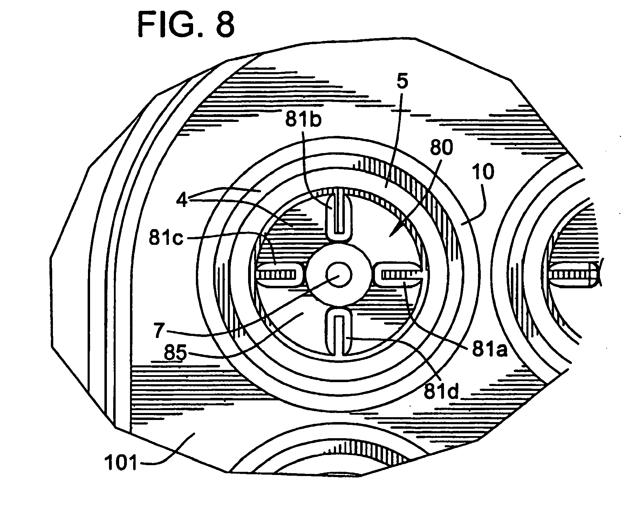

Typically, the device, and more typically, the well, includes one or more additional components for more efficient fluid processing, e.g., to prevent masking or blocking of the filter. For example, using the illustrative embodiments shown in Figures 3 and 8 (described below) for reference, the bottom end can include a drainage grid comprising one or more spacers and/or channels arranged such that fluid passing through the filter passes along the drainage channels between the grid spacers and then through the fluid flow port.

In a preferred embodiment, the bottom end comprises a drainage grid comprising at least one, more preferably, at least two, and even more preferably, at least three, drainage grid spacers projecting upwardly from the bottom wall. Illustratively, as shown in Figure 5a (cut away view), and shown in more detail in Figure 3 (top view), the bottom end 3 comprises a drainage grid 80 comprising a drainage grid wall 85 (a portion of bottom wall 4), drainage grid spacers 81a-d, 83a-d and 84a-d projecting upwardly from bottom wall 4, and drainage channels between the grid spacers (the drainage grid wall 85 forming the bottom of the drainage channels), wherein the drainage channels communicate with fluid flow port 7. Drainage grid spacers 81 a-d, 83a-d, and 84a-d, have top surfaces 82a-d, 86a-d, and 87a-d, respectively.

The drainage grid can have a variety of configurations so long as the filter can be supporter and sufficient space is allowed for the passage of filtrate. For example, in one embodiment (not shown), the spacers and/or channels can have a radial and/or circular configuration. Additionally, or alternatively, the drainage grid wall, the drainage channels and/or the drainage grid spacers can have, for example, a configuration sloping toward the fluid flow port 7. Fluid flow port 7 can have a tapered configuration if desired.

In some embodiments, e.g., using the embodiment illustrated in Figure 2 for reference, the top surfaces of the drainage grid spacers (e.g., top surfaces 82a and 82c, that can be planar or non-planar) are substantially coplanar with the bottom wall 4 between the circumferential rib 5 and the inner surface 2 of the tube. If desired, this portion of the bottom wall (between the rib and the inner surface of the tube) can be substantially coplanar with the surface of the drainage grid wall 85, and other configurations can also be suitable.

The tubular wall is preferably cylindrical, but in some embodiments includes multiple sides, e.g., multiple flat sides in order to provide a pentagonal, hexagonal, heptagonal, or octagonal shape, or a combination of flat and rounded portions.

While the tubular wall of the well can include a tapered section, e.g., a reduction in the inner diameter of the well along a portion of the length of the wall, the wall is preferably not tapered at the location where the sealing ring is retained against the inner surface of the side wall.

The sealing ring is preferably arranged to be frictionally engaged against the inner surface of the side wall. Since the tubular wall is preferably cylindrical, the sealing ring 40 preferably comprises a generally annular ring, the sealing ring having an axis, and an outer surface 43.

In the embodiment illustrated in Figures 4b, 5a and 5b, the sealing ring also comprises a bottom surface 41. More preferably, the bottom surface 41 has a width dimension radially of the ring that is generally the same as the radial width of the top surface 6 of the circumferential rib 5. Since the sealing ring 40 is preferably frictionally engaged against the inner wall of the well at position 30, the outer surface 43 of the sealing ring before insertion in the well has a diameter that is larger than the inner diameter of the wall of the well at position 30, e.g., the outer diameter of the sealing ring is compressed during insertion into the well. The ring, once inserted into the well, is retained against the inner surface of the wall with the bottom surface 41 of the ring pressing against the upper surface 21 of the filter 20, and pressing the peripheral portion of the lower surface 22 of the filter 20 against the top surface 6 of the rib arrangement 5. Accordingly, the filter can be efficiently secured and sealed in the well without utilizing welding and/or adhesives, e.g., to secure the sealing ring in the well.

In accordance with some preferred embodiments of the invention, e.g., as described with respect to Figure 5b above, and with respect to Figure 6 below, clamping and sealing forces can be concentrated on the portion of the filter contacting the bottom surface of the sealing ring and the portion of the filter contacting the top surface of the rib In addition, since the sealing ring in accordance with each of these embodiments is preferably retained at a location in the well wherein the wall of the well is not tapered, the preferred configuration provides an efficient seal, keeps the filter substantially flat, and provides for maintaining the seal during exposure to conditions (occurring, for example, during shipping and/or storage) that could cause conventional seals to "back out" of the wells, e.g., conditions such as vibration and changes in temperature.

In these embodiments, the rib typically becomes partially deformed when the seal is created in accordance with the invention. Since there is a range of achievable deformation while providing efficient sealing, the rib compensates for some variability in, for example, the filters and/or the plates (e.g., with-respect to thickness and/or flatness) of the devices. Additionally, in those embodiments including a plurality of separate inserts, the use of separate inserts allows the seal to be optimized with respect to individual wells and/or a series of wells, that also compensates for variability in the filters and/or plates.

The sealing ring can be made of any suitable material that provides a desired property or combination of properties, such as, for example, resilience, chemical compatibility with the sample or reaction components, and cost. Exemplary materials include, but are not limited to, rubber, silicone, and thermoplastic materials such as, for example, polypropylene and polystyrene.

As noted above, the device can have additional components. For example, the embodiment of the device illustrated in Figure 5b includes an outlet 200 (e.g., forming a nozzle) communicating with fluid flow port 7. The outlet can have any suitable dimension and/or configuration. If desired, the outlet can be arranged to further reduce undesired liquid loss from the well caused by capillary action and/or gravity flow (sometimes referred to as "wricking). For example, the outlet can include a shoulder or collar preventing liquid from migrating upwardly along the outer diameter of the outlet. Alternatively, or additionally, the outlet can, for example, have any desired length. Illustratively, in the embodiment shown in Figure 5b, the tip of the outlet does not extend to the bottom surface of plate 101. In other embodiments, e.g., as shown in Figure 6, the tip of the outlet extends beyond the bottom surface of plate 101 (Figure 6). Typically, a longer outlet (e.g., as shown in Figure 6) is desirable for those applications wherein the filtrate is to be analyzed, and the tip of the outlet will extend into a receiving plate during use. Alternatively, a shorter outlet (e.g., as shown in Figure 5b) can be desirable for those applications wherein the retentate (the material retained in the well) is to be analyzed, as the tip of the outlet will not contact the surface of, for example, the lab bench when the plate 101 is placed on the lab bench. Thus, the potential for contamination of the work area and/or the filtrate can be reduced.

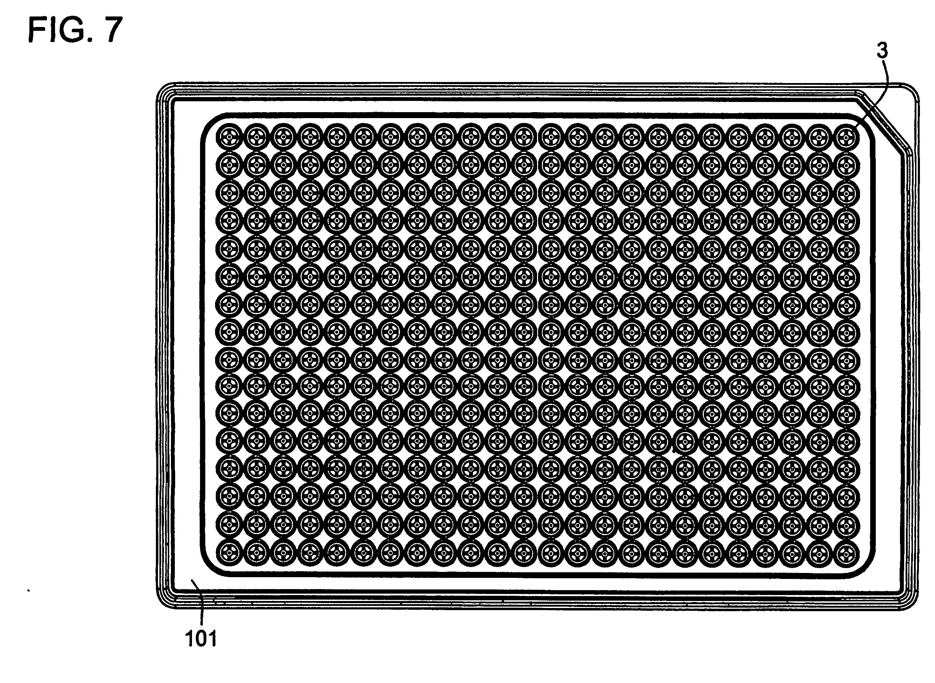

A plurality of wells can be connected together, e.g., in the form of a strip, disc, sheet, or tray. In preferred embodiments, a plurality of wells are connected together for use in devices according to the invention. For example, Figure 1 and 7 show portions of embodiments of devices providing 96 well and 384 well devices. In another illustrative embodiment (not shown) a plurality of wells connected in the form of one or more strips can be attached (e.g., by a snap fit) to a support, frame, or plate. Alternatively, or additionally, a plurality of inserts can be connected together, for example, in the form of a strip, disc, sheet, or tray.

In some embodiments of the invention, especially some embodiments of multiple well devices according to the invention, the device comprises at least one plate or tray defining at least a part of the upper portion of the wells or at least a part of the lower portion of the wells (the plate or tray can comprise the sealing ring); a plurality of separate components (preferably inserts) defining at least a part of another portion of the well (e.g., a part of the lower portion wherein the plate or tray defines at least a part of the upper potion of the wells; and the separate insert can further comprise the sealing ring), and a filter compressed by the sealing ring. In other embodiments of multiple well devices, the device comprises at least a top plate and a bottom plate, and a filter. For example, the device can comprise an upper plate or tray defining the upper portion of the wells and/or the sealing ring, a bottom plate or tray defining at least the bottom wall of the wells, and a filter between the sealing ring and the bottom wall. Typically, the plates and/or separate components are snap-fit or press-fit together. In some embodiments, the plates and/or separate components are snap-fit or press-fit together and subsequently additionally bonded, e.g., via welding (such as ultrasonic welding), adhesives and/or solvents.

In accordance with the embodiment illustrated in partial cross-sectional view in Figure 6, multiple well device 100 comprises a top plate 102 comprising sealing rings 40, a bottom plate 101 comprising bottom walls 4, and filters 20 each comprising at least one filter element 25, between the sealing rings and the bottom walls. The Figure shows two wells 50 (one well in exploded view), each well including a filter 20 comprising at least one filter element 25. The bottom plate 101 comprises (for each well) a side wall 10 having an inner surface 2, bottom end 3 comprising a bottom wall 4, a circumferential rib 5 and a fluid flow port 7, and a top plate 102 comprises (for each well) a sealing ring 40 having a bore 45. In the illustrated assembled well, the filter is compressed between the bottom surface 41 of the sealing ring and the top surface 6 of the rib.

As with the embodiment illustrated in Figures 1-5, the circumferential rib 5 illustrated in Figure 6 has a top surface 6 (that is preferably a non-planar surface) disposed to contact the lower surface 22 of the filter 20 comprising at least one filter element 25. The top surface 6 of the rib does not contact the inner surface 2 of the side wall of the well, and the rib can be located a predetermined distance from the inner surface of the side wall.

In accordance with the embodiment illustrated in Figure 6, wherein the top plate 102 comprises a sealing ring 40, the ring, having a bottom surface 41, and an outer surface 43, is retained against the inner surface of the hollow tubular wall, the outer surface 43 of the ring pressing against the inner surface 2 of the side wall (e.g., at location 30), and the lower surface 41 of the ring pressing against the upper surface 21 of the filter 20 while the top surface 6 of the rib 5 presses against the lower surface of the filter. In this embodiment, as with the embodiments illustrated in Figures 1-5, since the sealing ring 40 efficiently seals the filter 20 in the well; fluid is prevented from bypassing the filter.

Figures 7-10 illustrate the individual components of the multiple well device 100 including the assembled well 50 shown in Figure 6. Accordingly, Figure 7 shows a top view of the bottom plate 101, showing the bottom ends of a plurality of wells, and Figure 8 shows a top view of the bottom end of a well in more detail, wherein the end 3 includes a bottom wall 4, a circumferential rib 5 projecting upwardly from the bottom wall, a drainage grid 80 comprising a plurality of drainage grid spacers 81a-d projecting upwardly from the drainage grid wall 85 (a portion of bottom wall 4), and a fluid flow port 7. However, in other embodiments of the invention, the lower portion of the wells can have different arrangements, e.g., as shown in Figures 1 and 3.

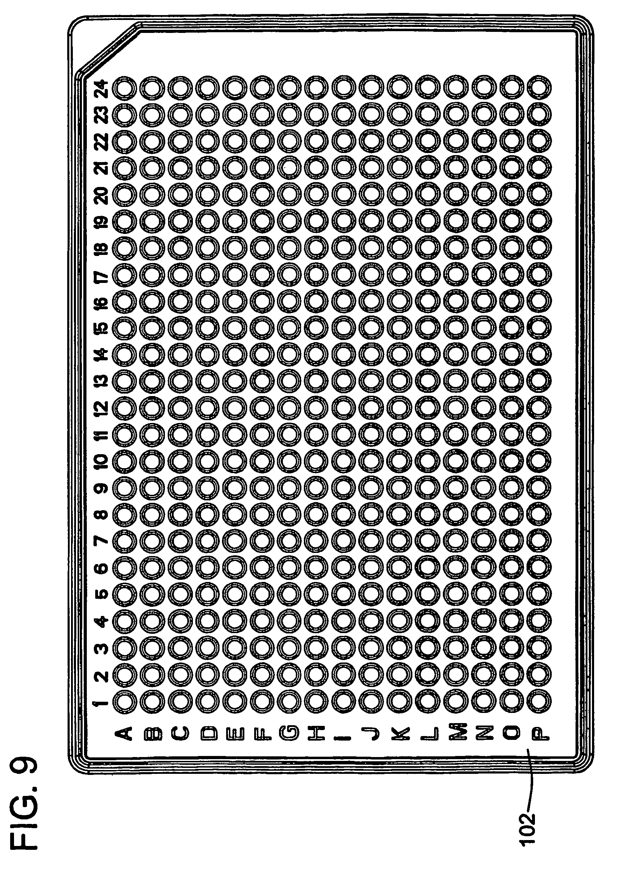

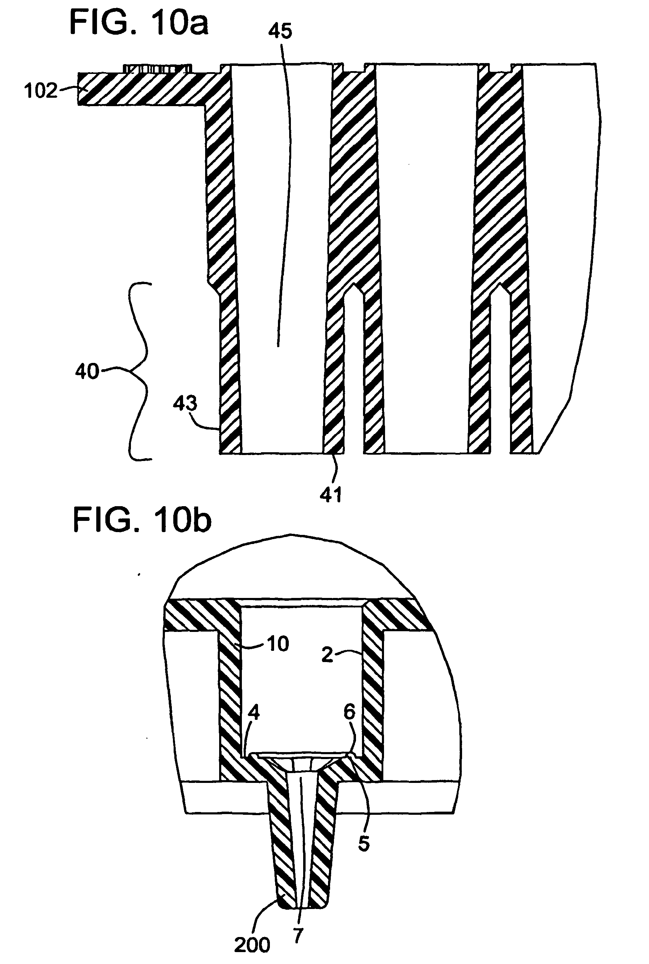

Figures 9 and 10a show, respectively, a top view, and a-partial cross-sectional view of the top plate 102 shown in Figure 6, and Figure 10b shows a partial cross-sectional view of the bottom plate 101. As shown in more detail in Figure 10a, top plate 102 comprises a sealing ring 40 having a bore 45, the ring having an outer surface 43 and a lower surface 41. The outer surface 43 of the ring presses against (as shown in Figure 6) the inner surface 2 of the side wall of the tube 10 in the bottom plate 101.

In the embodiment illustrated in Figures 6 and 10a, the sealing ring 40 has a bore 45, and the bore extends to the top surface of the top plate. The bore is suitable for receiving fluid, and typically (using Figure 6 for reference), fluid does not contact the inner surface 2 of the side wall 10. This is in contrast with, for example, the embodiments illustrated in Figure 5, wherein fluid can contact the inner surface 2.

Embodiments of devices according to the invention can include a separate sealing ring (e.g., as shown in Figure 5), or a sealing ring as part of an upper portion of the device (e.g., top plate 102 as shown in Figures 6 and 10a).

As described above with respect to embodiments of the invention, the stealing ring 40 is preferably frictionally engaged against the inner wall of the well, while providing for sealing the filter in the well without requiring the use of welding and/or adhesives to retain the ring in the well. For example, using the embodiments illustrated in Figures 5b and 6 for reference, the sealing ring is frictionally engaged against the wall at location 30, with the bottom surface 41 of the ring pressing against the upper surface 21 of the filter 20, and the peripheral portion of the lower surface 22 of the filter 20 pressing against the top surface 6 of the circumferential rib 5. The sealing ring can also be frictionally engaged against the wall at position 30. If desired, position 30 can be predetermined, e.g., by adjusting the thickness and/or density of the filter elements. Alternatively, or additionally, the position can be predetermined by controlling the force applied to introduce the sealing ring into the hollow tube (Figure 5) or the force applied to introduce the inserts into the trays. The frictional engagement is preferably accomplished by adjusting the relative sizes of the outer diameter of the sealing ring and the inner diameter of the well.

A variety of materials are suitable for producing ribs, lips, drainage grids, inserts, chambers, plates, and sealing rings according to the invention. Illustrative materials include, for example, polyvinyl chloride with or without copolymers, polyethylenes, polystyrenes, polystyrene-acrylonitrile, polypropylene, polyvinylidine chloride, and the like.

The wells and multiple well devices according to the invention can have any suitable overall dimension and capacity, although preferred embodiments have substantially the same overall dimension and capacity of standard wells and devices. Such embodiments are preferred as being more easily utilized with devices and instruments, such as liquid handling systems and readers, that are commonly available for use with conventional wells and devices. In those applications wherein a signal is generated, e.g., in the course of a microtitration assay, the signal can be read by any suitable means, e.g., by visual analysis, or the detection of a fluorometric, spectrophotometric, radiometric, or chemiluminescent signal.

Typically, each well is suitable for receiving at least about 200 microliters (µL), more typically, at least about 350 µL, of fluid to be processed. In some embodiments, each well is suitable for receiving at least about 800 µL of fluid, or at least about 1 mL, or at least about 2 mL of fluid, or more.

If desired, the well, more typically, a multiple well device, is chemically resistant, and can be used with harsh solvents and/or harsh chemicals.

The present invention is useful in a variety of applications including ' microtitration, microchromatography, radiography, microfiltration, ultrafiltration, nanofiltration, washing processes, polymerase chain reaction (PCR) analysis, high throughput, especially high throughput screening (HTS), combinatorial chemistry, nucleic acid and protein processing (including synthesis, sequencing, separation and/or purification) and microculture of cell suspensions and tissues. The invention can be used in any suitable setting, including, but not limited to, hospitals and laboratories. Embodiments of the invention are suitable for a variety of protocols, including sample preparation, clinical diagnostic assays, and screening specimens, e.g., drugs in pharmaceutical research. If desired, materials (e.g., ligands, nucleic acids) can be bound to solid phase particles such as beads and collected in the wells, and the materials can be cleaved from the particles such that the materials are collected in the filtrate. In some embodiments, the cleaved materials (e.g., ligands and synthesized nucleic acids) can be further processed (e.g., screened) using another well, e.g., the filtrate can be passed into one or more multiple well devices. Alternatively, or additionally, the invention can be compatible with other subsequent processes including, but not limited to, at least one of dot blotting, immunoblotting, receptor binding assays, ELISA, and RIA.

Embodiments of the invention are especially suitable for removing particulates from a fluid, e.g., to provide a filtered sample for analysis, for example, by high pressure liquid chromatography (HPLC), gas chromatography (GC), mass spectrometry (MS), infra red (IR), nuclear magnetic resonance (NMR), and solid-phase extraction.

Accordingly, a variety of filters and filter elements (as used herein, the terms "filter" and "filter element" refer to porous media used in these various applications) are suitable for use in the invention, and those skilled in the art will recognize that the choice of filter(s) and filter element(s) will depend on the intended use of the well, The filter can comprise a depth filter and/or a sieve filter. The filter can include fibrous and/or membrane filter elements. The filter can include additional elements and/or components such as, for example, at least one of a drainage, cushion, and prefilter layer. Typical filter elements include membranes, especially polymeric membranes. For some applications, the filter elements are chemically resistant, and can be used with harsh solvents and/or harsh chemicals. In some embodiments, the filter comprises a plurality of filter elements, and the elements can have different characteristics, e.g., at least one of pore size, chemistry (for example, at least one of critical wetting surface tension, surface charge, polarity, hydrophilicity, and attached functional groups), and can include different reagents and assay components.

Filters and filter elements of suitable shape for use in the invention are typically stamped or otherwise cut out of sheets of suitable material(s), e.g., membrane sheets.

A filter element such as a porous membrane or a fibrous element can have any suitable pore structure such as a pore size, or a pore rating or a pore diameter. Thus, e.g., a filter element comprising a membrane typically has an average pore size of about 100 µm or less, preferably from about 0.01 µm to about 100 µm. In some embodiments, the membrane has an average pore size of about 0.1 µmn or less, or from about 0.1 µm to about 10 µm. Preferably, the membrane has an average pore size of about 5 µm or less.

The use of the terms "a" and "an" and "the" and similar referents in the context of describing the invention (especially in the context of the following claims) are to be construed to cover both the singular and the plural, unless otherwise indicated herein or clearly contradicted by context. Recitation of ranges of values herein are merely intended to serve as a shorthand method of referring individually to each separate value falling within the range, unless otherwise indicated herein, and each separate value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein, is intended merely to better illuminate the invention and does not pose a limitation on the scope of the invention unless otherwise claimed. No language in the specification should be construed as indicating any non-claimed element as essential to the practice of the invention.

## Claims

1. A multiple well device comprising:
a plurality of wells for receiving liquid samples to be processed, each well having a side wall having an inner surface, a bottom end comprising a bottom wall and a fluid flow port;
a filter comprising at least one filter element in each well, the filter having an upper surface and a lower surface; and
a sealing ring having a bottom surface pressing against the upper surface of the filter for sealing same, **characterized in that** each well comprises a circumferential rib projecting upwardly from the bottom wall, the circumferential rib having a top surface spaced from the inner surface of the side wall and pressing against the lower surface of the filter, said sealing ring having an outer surface and a bottom surface, the bottom surface having a width that is at least the width of the top surface of the circumferential rib, said sealing ring sealing the filter against the circumferential rib and pressing against the inner surface of the side wall.

2. The well device of claim 1, wherein the filter comprises a membrane.

3. The well device of claim 2, wherein the membrane comprises a microporous membrane.

4. The well device of claim 3, wherein the membrane comprises an ultrafiltration membrane.

5. The well device of claim 1, wherein the filter comprises a fibrous medium.

6. The well device of any of claims 1 to 5, wherein the filter comprises at least two filter elements.

7. The well device of any of claims 1 to 6, wherein the side wall is not tapered where the sealing ring presses against the inner surface of the side wall.

8. The well device of claim 1 or 2, wherein the lower end includes a drainage grid.

9. The well device of any one of claims 1 to 8, comprising 96 wells.

10. The well device of any one of claims 1 to 8, comprising 384 wells.

11. A method for processing a fluid comprising:
passing a fluid into a well of a multiple well device comprising a plurality of wells, each well comprising a side wall having an inner surface and a bottom end having a fluid flow port; the well having a filter comprising at least one filter element sealed therein; and
passing at least a portion of the fluid through the filter and through the fluid flow port at the bottom end of the well, **characterized in that** each bottom end has a circumferential rib having a top surface, wherein the top surface of the circumferential rib is spaced from the inner surface of the side wall and the filter being compressed between a lower surface of a sealing ring, and the top surface of the circumferential rib.

12. The method of claim 11, wherein passing the fluid through the filter includes depleting the fluid of particulates.

13. The method of claims 11 or 12, further comprising analyzing the fluid passing through the filter.

## Patentansprüche

1. Multiwell-Vorrichtung, umfassend:
eine Mehrzahl von Wells zur Aufnahme von zu behandelnden Flüssigproben, wobei jedes Weil eine Seitenwand mit einer inneren Oberfläche, ein Bodenende umfassend eine Bodenwand und eine Fluiddurchflussöffnung aufweist;
ein Filter, umfassend mindestens ein Filterelement in jedem Well, wobei das Filter eine obere Oberfläche und eine untere Oberfläche aufweist; und
einen Dichtring mit einer gegen die obere Oberfläche des Filters drückenden Bodenfläche zum Dichten desselben, **dadurch gekennzeichnet, dass** jedes Well eine von der Bodenwand nach oben abstehende Umfangsrippe umfasst, wobei die Umfangsrippe eine obere Oberfläche aufweist, welche von der inneren Oberfläche der Seitenwand beabstandet ist und gegen die untere Oberfläche des Filters drückt, wobei der Dichtring eine äußere Oberfläche und eine Bodenfläche aufweist, wobei die Bodenfläche eine Breite aufweist, die mindestens die Breite der oberen Oberfläche der Umfangsrippe beträgt, wobei der Dichtring das Filter gegen die Umfangsrippe dichtet und gegen die innere Oberfläche der Seitenwand drückt.

2. Well-Vorrichtung nach Anspruch 1, wobei das Filter eine Membran umfasst.

3. Well-Vorrichtung nach Anspruch 2, wobei die Membran eine mikroporöse Membran umfasst.

4. Well-Vorrichtung nach Anspruch 3, wobei die Membran eine Ultrafiltrationsmembran umfasst.

5. Well-Vorrichtung nach Anspruch 1, wobei das Filter ein faseriges Medium umfasst.

6. Well-Vorrichtung nach einem der Ansprüche 1 bis 5, wobei das Filter mindestens zwei Filterelemente umfasst.

7. Well-Vorrichtung nach einem der Ansprüche 1 bis 6, wobei die Seitenwand nicht verjüngt ist, wo der Dichtring gegen die innere Oberfläche der Seitenwand drückt.

8. Well-Vorrichtung nach Anspruch 1 oder 2, wobei das untere Ende ein Drainagegitter umfasst.

9. Well-Vorrichtung nach einem der Ansprüche 1 bis 8, umfassend 96 Wells.

10. Well-Vorrichtung nach einem der Ansprüche 1 bis 8, umfassend 384 Wells.

11. Verfahren zum Behandeln eines Fluids, umfassend:
Einleiten eines Fluids in ein Well einer Multiwell-Vorrichtung, welche eine Mehrzahl von Wells umfasst, wobei jedes Well eine Seitenwand mit einer inneren Oberfläche und ein Bodenende mit einer Fluiddurchflussöffnung umfasst; wobei das Well ein Filter aufweist, umfassend mindestens ein Filterelement, welches darin gedichtet ist; und
Durchleiten von mindestens einem Teil des Fluids durch das Filter und durch die Fluiddurchflussöffnung an dem Bodenende des Wells hindurch, **dadurch gekennzeichnet, dass** jedes Bodenende eine Umfangsrippe mit einer oberen Oberfläche aufweist, wobei die obere Oberfläche der Umfangsrippe von der inneren Oberfläche der Seitenwand beabstandet ist und das Filter zwischen einer unteren Oberfläche eines Dichtrings und der oberen Oberfläche der Umfangsrippe zusammengedrückt wird.

12. Verfahren nach Anspruch 11, wobei das Hindurchleiten des Fluids durch das Filter umfasst, das Fluid an Partikeln zu verarmen.

13. Well-Vorrichtung nach Anspruch 11 oder 12, ferner umfassend das Analysieren des durch das Filter hindurch geleiteten Fluids.

## Revendications

1. Dispositif à puits multiples, comprenant :
une pluralité de puits pour recevoir des échantillons de liquide à traiter, chaque puits ayant une paroi latérale ayant une surface intérieure, une extrémité de fond comprenant une paroi de fond et un orifice d'écoulement de fluide ;
un filtre comprenant au moins un élément filtrant dans chaque puits, le filtre ayant une surface supérieure et une surface inférieure ; et
une bague d'étanchement ayant une surface de fond qui presse contre la surface supérieure du filtre pour étancher celui-ci,
**caractérisé en ce que** chaque puits comprend une nervure circonférentielle qui se projette vers le haut depuis la paroi de fond, la nervure circonférentielle ayant une surface supérieure espacée de la surface intérieure de la paroi latérale et pressant contre la surface inférieure du filtre, ladite bague d'étanchement ayant une surface extérieure et une surface de fond, la surface de fond ayant une largeur qui est au moins la largeur de la surface supérieure de la nervure circonférentielle, ladite bague d'étanchement étanchant le filtre contre la nervure circonférentielle et pressant contre la surface intérieure de la paroi latérale.

2. Dispositif à puits multiples selon la revendication 1, dans lequel le filtre comprend une membrane.

3. Dispositif à puits multiples selon la revendication 2, dans lequel la membrane comprend une membrane microporeuse.

4. Dispositif à puits multiples selon la revendication 3, dans lequel la membrane comprend une membrane d'ultrafiltration.

5. Dispositif à puits multiples selon la revendication 1, dans lequel le filtre comprend un média fibreux.

6. Dispositif à puits multiples selon l'une quelconque des revendications 1 à 5, dans lequel le filtre comprend au moins deux éléments de filtre.

7. Dispositif à puits multiples selon l'une quelconque des revendications 1 à 6, dans lequel la paroi latérale n'est pas effilée à l'endroit où la bague d'étanchement presse contre la surface intérieure de la paroi latérale.

8. Dispositif à puits multiples selon la revendication 1 ou 2, dans lequel l'extrémité inférieure inclut une grille de drainage.

9. Dispositif à puits multiples selon l'une quelconque des revendications 1 à 8, comprenant 96 puits.

10. Dispositif à puits multiples selon l'une quelconque des revendications 1 à 8, comprenant 3 84 puits.

11. Procédé pour traiter un fluide, comprenant les étapes :
faire passer un fluide dans un puits d'un dispositif à puits multiples comprenant une pluralité de puits, chaque puits comprenant une paroi latérale ayant une surface intérieure et une extrémité de fond ayant un orifice d'écoulement de fluide ; le puits ayant un filtre comprenant au moins un élément de filtre scellé à l'intérieur ; et
faire passer au moins une portion du fluide à travers le filtre et à travers l'orifice d'écoulement de fluide à l'extrémité de fond du puits,
**caractérisé en ce que** chaque extrémité de fond possède une nervure circonférentielle ayant une surface supérieure, ladite surface supérieure de la nervure circonférentielle étant espacée de la surface intérieure de la paroi latérale et le filtre étant comprimé entre une surface inférieure d'une bague d'étanchement et la surface supérieure de la nervure circonférentielle.

12. Procédé selon la revendication 11, dans lequel l'étape consistant à faire passer le fluide à travers le filtre inclut de débarrasser le fluide de ses particules.

13. Procédé selon les revendications 11 ou 12, comprenant en outre d'analyser le fluide qui passe à travers le filtre.
